# EUROPEAN PATENT SPECIFICATION

(11) **EP 4 210 629 B1**
(45) Date of publication and mention of the grant of the patent: **01.07.2026**
(21) Application number: 21766527.2
(22) Date of filing: 08.09.2021
(51) Int. Cl.: A61F 2/00, A61F 2/08, A61L 27/56

(54) **DEVICE FOR INTERFACING FILAMENTOUS OR FIBROUS STRUCTURES WITH A REAL OR SIMULATED BIOLOGICAL TISSUE**
VORRICHTUNG ZUR KOPPLUNG VON FADENFÖRMIGEN ODER FASERIGEN STRUKTUREN MIT EINEM REALEN ODER SIMULIERTEN BIOLOGISCHEN GEWEBE
DISPOSITIF D'INTERFAÇAGE DE STRUCTURES FILAMENTEUSES OU FIBREUSES AVEC UN TISSU BIOLOGIQUE RÉEL OU SIMULÉ

(30) Priority: 09.09.2020 IT 202000021313
(43) Date of publication of application: 19.07.2023
(73) Proprietor: Alma Mater Studiorum Universita di Bologna, 40126 Bologna (IT)
(72) Inventor: SENSINI, Alberto, 40126 Bologna (IT); ZUCCHELLI, Andrea, 40126 Bologna (IT); CRISTOFOLINI, Luca, 40126 Bologna (IT); GUALANDI, Chiara, 40126 Bologna (IT); FOCARETE, Maria Letizia, 40126 Bologna (IT)
(74) Representative: Praxi Intellectual Property Milano
(86) International application number: PCT/IB2021/058153
(87) International publication number: WO 2022/053940

(56) References cited:
- EP-A2- 0 260 970
- US-A1- 2005 112 397
- US-A1- 2016 100 932
- GOSWAMI DEBKALPA ET AL: "3D-Architected Soft Machines with Topologically Encoded Motion", ADVANCED FUNCTIONAL MATERIALS, vol. 29, no. 24, 10 June 2019 (2019-06-10), DE, pages 1808713, XP055806608, ISSN: 1616-301X, DOI: 10.1002/adfm.201808713

## Description

### TECHNICAL FIELD OF THE INVENTION

The present invention relates to a device for interfacing filamentous, or fibrous, structures with a real or simulated biological tissue. For the purposes of the present description, the term "filamentous or fibrous structures" means any structure comprising one or more filaments or fibers. Preferably, such filamentous structures, are hierarchical electrospun supports for regeneration, or repair, or replacement, of tendon/ligamentous tissue that, by means of the device of the present invention, can be interfaced with a real biological tissue, such as bone tissue. Therefore, the present invention further relates to a system for regeneration, or repair, or replacement, of tendon/ligamentous tissue comprising such electrospun hierarchical supports anchored to the device for interfacing.

Such filamentous structures, may, in addition, be electrospun hierarchical supports for the simulation of tendon/ligamentous and/or muscle tissue which, thanks to the device of the present invention, are interfaced, with a simulated biological tissue, in order to obtain parts of robotic systems for the simulation of the mechanical behavior of muscles such as, for example, actuators of a prosthesis. Thus, the present invention also relates to a system for simulating tendon/ligamentous and/or muscle tissue comprising such electrospun hierarchical supports anchored to the device for interfacing, as well as to a prosthesis actuator or robotic system comprising such a system.

### STATE OF THE ART

At the present state of the art, in the field of tissue engineering, supports are known for cell adhesion, proliferation and migration, whose morphology is fundamental for the final shape and structure of the tissues and organs to be reconstructed or replaced.

Such supports, are also commonly known as "scaffolds". In particular, in the field of ligament or tendon reconstruction, "scaffolds" consisting of bundles formed by nanofibers obtained by electrospinning are known. Such bundles are arranged in groups joined together, then, so as to form a single bundle and are, usually, covered by a porous membrane that keeps them aligned and compacts them (WO2018229615 A1). Such a membrane is then, in turn, formed of nanofibers. The "scaffolds" for the reconstruction of tendons and ligaments are therefore filamentous or fibrous structures that need to be anchored in vivo to the bone tissue on which the tendon/ligament to be reconstructed must be grafted. Similarly, there is also the need to anchor these filamentous or fibrous "scaffolds" to simulated biological tissue, such as, for example, that used in parts of robotic systems for the simulation of the mechanical behavior of muscles (e.g. actuators of prostheses) or of the interface between muscle tendon and bone.

In the field of tendon and ligament reconstruction, devices are currently known comprising, essentially, two elements: a screw intended to be grafted into bone tissue and an anchoring element of any artificial tendon or ligament configured to be embedded in the screw (US 2013/090731 A1). EP0260970A2 also discloses a device for fixing tendons/ligament (filamentous structure) to a bone, comprising a porous grommet configured for the wrapping of the filamentous structure. US2005/112397, instead, discloses a porous structure having a plurality of bonded sheets, each sheet having at least one aperture that partially overlaps an aperture of at least one other sheet. Said porous structure can be used to build bone substitute component of an orthopedic implant. Such devices of the known art may be suitable for biological grafts or prostheses with artificial materials, but are not suitable for anchoring "scaffolds" for cellular regeneration. The latter, in fact, are supports on which, the cells that are deposited, must multiply and differentiate, depending on the tissue that is intended to regenerate, repair, or simulate. In the specific case, the cells deposited on the same "scaffold" must differentiate into fibroblasts (e.g. cells of connective tissue that makes up the tendons and ligaments) and osteoblasts (e.g. cells of bone tissue). To achieve such differentiation, with a single "scaffold", it is necessary to reproduce, simultaneously, different mechanical conditions, or rather, different degrees of strain.

These, in turn, produce different degrees of mineralization and, therefore, cells of different types. In fact, in nature, in the transition between tendon and bone, there is a gradient of deformability that is at the origin of the different degrees of mineralization of the cells, which differentiate into fibroblasts of the tendon where they are subjected to the maximum strain, into fibroblasts of the thin layer of mineralized fibrocartilage that interfaces between tendon and bone, up to osteoblasts of the bone tissue where they are subjected to the minimum strain. The devices known to the state of the art mentioned above are not able to ensure any gradient of deformability and, therefore, even if they are attached to "scaffolds" for tendon regeneration, they are not able to obtain the cell differentiation described above.

### OBJECTS AND SUMMARY OF THE INVENTION

The object of the present invention is, therefore, to provide a device for interfacing filamentous, or fibrous, structures with a real or simulated biological tissue that mimics the mechanical characteristics of the tendon/ligament/bone interface.

More particularly, the object of the present invention is to provide a device for interfacing supports or "scaffolds" for the regeneration, repair, of tendons or ligaments that, once implanted ensures a gradient of deformability such that the cells, deposited on said "scaffold", will differentiate into the cell types characteristic of: (1) tendon/ligament tissue; (2) fibrocartilage tissue at the tendon/bone interface; and (3) bone tissue, thereby achieving complete integration of the tendon-muscle and/or ligament system to the bone.

This object is achieved by designing the device of the present invention such that it has porous zones having a trabecular structure. The devices according to the present invention are as defined in claims 1 and 2.

For the purposes of the present invention, the term "trabecular structure" means a structure that mimics the trabecular structure of spongy bone. As is known, the latter is, in fact, a tissue consisting of thin columnar structures or trabeculae, variously oriented and intertwined with each other to delimit numerous intercommunicating cavities called areoles or medullary cavities containing bone marrow, blood vessels and nerves.

To achieve the aforementioned object, the present invention provides a device as defined in claim 1. The trabecular structure allows to mimic the structure of the bone tissue in order to better simulate the mechanical characteristics of the tendon/ligament/bone interface.

This interface is even better simulated by providing that the body for anchoring the filamentous structure comprises, not a single porous zone with homogeneous porosity, but, at least a first porous zone and a second porous zone, having different degrees of porosity.

For the purposes of the present invention, the term porosity means the percentage ratio of the total volume of pores (or voids) to the total volume of the body or material considered.

More particularly, the body for anchoring the filamentous structure may be formed in the guise of tweezer with two flat, porous arms joined to each other by means of the capstan. In such a case, the second body consists of a screw with a threaded surface. As will be explained in more detail below, the tweezer are interlocked inside the screw.

Since the tweezer and the screw differ, between them, both from the point of view of shape and porosity, they will be subjected to different strains, so as to simulate that gradient that, in nature, is created in the passage between tendon and bone, or more specifically in that layer of cartilage that is interposed between the tendon or ligament and the bone.

The tweezer-screw assembly may be, in particular, intended, preferably but not exclusively, for regeneration, or repair, or replacement, as well as simulation of a tendon or cylindrical ligament. In such a case, the implantation of the interface device, or rather of the system constituted by the device and the filamentous structure itself, may take place in the following way: the health care operator (e.g. surgeon) implants the screw in the bone and then inserts interlockingly, inside the screw, the tweezer, around whose capstan is wound the filamentous structure which, in this case will be, a tendon and/or ligament scaffold constituted by a bundle of nanofibers obtained by electrospinning.

A second object of the present invention is, therefore, also to provide a device for interfacing filamentous, or fibrous structures with a real biological tissue that allows a certain ease and safety in the implantation operations of the filamentous structure into the bone tissue in vivo. If the device were in fact made of a single element such as, for example, a screw, the surgeon would have to first fix the filamentous structure inside the screw and then screw the latter to the patient's bone. In this way, in addition to the fact of having to perform an uncomfortable procedure, there would be the risk of damaging the filamentous structure itself, compromising its operation. On the contrary, with the present invention, since the object being implanted consists only of the screw without any filamentous structure inside it, the operation of fixing the screw in the bone does not compromise, in any way, the integrity of the filamentous structure. The operation of interlocking the tweezer with the filamentous structure wrapped around its capstan is, in fact, less risky for the integrity of the filamentous structure than the operation of inserting the screw into the bone.

Alternatively, to the assembly comprising the tweezer and the screw, the body for anchoring the filamentous structure and, therefore, the device itself forming the subject of the present invention may comprise a plate having a plurality of capstans configured for wrapping the filamentous structure.

Said plate may be, in particular, preferably but not exclusively intended for regeneration, or repair, or replacement, as well as simulation of tendons and/or flat ligaments such as, for example, those of the rotator cuff of the scapulo-humeral joint. This plate can also be used for the regeneration, repair, replacement of spinal ligaments. Also in this case, the plate can comprise areas of different porosity in order to generate gradients of deformability and, therefore, allow the differentiation of cells into different cell types, depending on the imposed strain. More particularly, in the plate of the present invention, it is envisaged that the zone farthest from the capstans, namely the zone farthest from the filamentous structure simulating the tendon(s)/link(s), has a higher porosity (e.g., larger pore size), than the zone in the proximity of the capstans (e.g., smaller pore size).

It is further disclosed herein that the present invention relates not only to a device for interfacing a filamentous structure, with real or simulated biological tissue, but also to a system for regenerating, or repairing, or replacing, or simulating tendon and/or ligament tissue comprising the above-described device and at least one filamentous structure. The latter may comprise a plurality of groups of nanofibers obtained by electrospinning, said plurality of groups being arranged to form a single bundle wrapped to the capstan (in the case of the tweezer), or wrapped to each of the capstans (in the case of the plate).

In this context, a third object of the present invention is, to, provide a system for regenerating, or repairing, or replacing, or simulating tendon and/or ligamentous tissue that promotes cell passage between nanofibers and bone without creating discontinuities in such passage. To this end, the system of the present invention may comprise nanofibers, or in general electrospun elements, within the porosity of the device, whether consisting of the tweezer alone, the screw- tweezer assembly, or the plate. In this way, no discontinuities are created between the various elements of the device (e.g., tweezer-screw assembly) and the tendon-cortical bone interface is better simulated.

These and further objects of the present invention will be made clearer by reading the following detailed description of some preferred embodiments to be understood purely as a not limiting example of the more general concepts claimed.

### BRIEF DESCRIPTION OF DRAWINGS

The following description refers to the attached drawings, wherein:
- Figure 1 is a perspective view of the first embodiment of the device of the present invention;
- Figure 2a is a front view of the first embodiment of the device of the present invention;
- Figure 2b is a side view of the first embodiment of the device of the present invention;
- Figure 3a is a first perspective view of a detail comprising the second body of the second embodiment of the device of the present invention;
- Figure 3b is a second perspective view of a detail constituted by the second body of the second embodiment of the device of the present invention;
- Figure 4 is a longitudinal section of a particular constituted by the second body of the second embodiment of the device of the present invention;
- Figure 5a is a first longitudinal section of the second embodiment of the device of the present invention;
- Figure 5b is a second longitudinal section of the second embodiment of the device of the present invention, said second longitudinal section being in a plane orthogonal to that of the first longitudinal section;
- Figure 6 is a perspective view of the fourth embodiment of the device of the present invention;
- Figure 7a is a front view of the fourth embodiment of the device of the present invention;
- Figure 7b is a front rear view of the fourth embodiment of the device of the present invention;
- Figure 8 is an exemplary schematic of a Voronoi trabecular tessellation followed by the porous portions of the device of the present invention;
- Figure 9a is a side section of a normal stress diagram obtained with a finite element model of the first embodiment of the system of the present invention;
- Figure 9b is a front section of a diagram of the equivalent Von Mises stresses obtained with a finite element model of a part of the first embodiment of the system of the present invention, said part being constituted by the body for anchoring the filament structure;
- Figure 10a is side section of a strain diagram obtained with a finite element model of the first embodiment of the system of the present invention; and
- Figure 10b is a side section of a strain diagram obtained with a finite element model of a part of the first embodiment of the system of the present invention, said part being constituted by the body for anchoring the filamentous structure and a bundle of nanofibers anchored to said body.

### DETAILED DESCRIPTION OF THE INVENTION

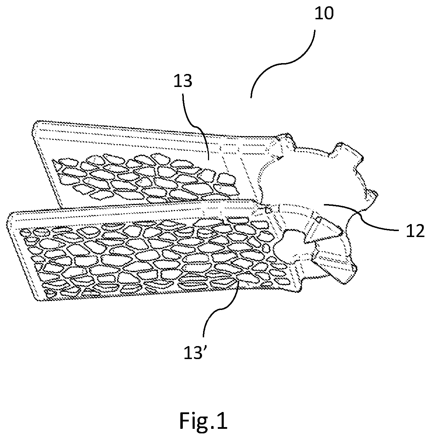

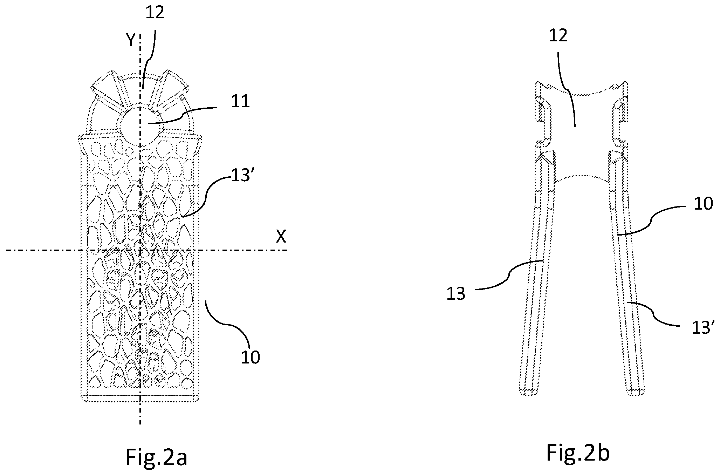

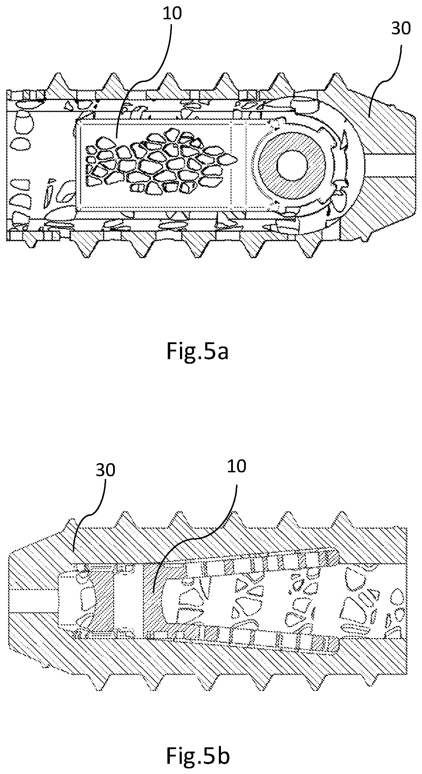

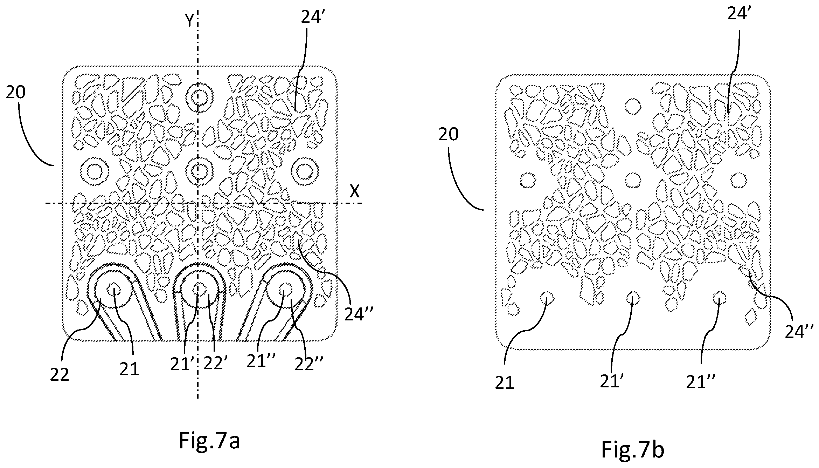

Referring to Figures 1, 2a, 2b, 5b, 6, 7a, 7b, the device for interfacing at least one filamentous structure, with real or simulated biological tissue, of the present invention, comprises at least one body (10, 20) for anchoring the filamentous structure, said body (10, 20) comprising:
- at least one capstan (12, 22, 22', 22') configured for wrapping the filamentous structure;
- at least one portion (13, 13', 24) porous having a trabecular structure (300).

In the center of the at least one capstan (12, 22, 22', 22") there may be a hole (11, 21, 21', 21").

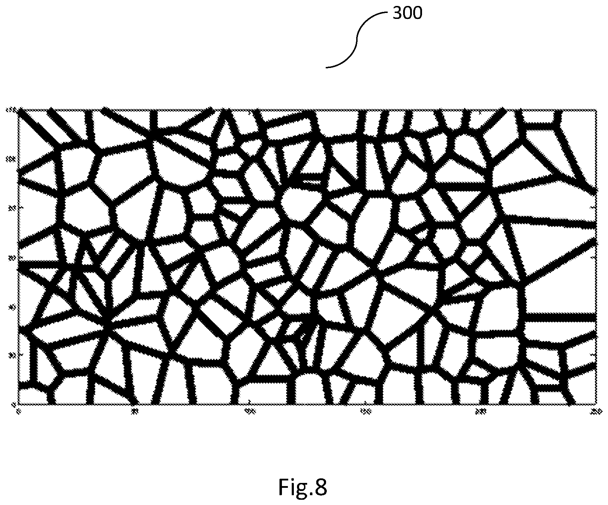

Referring to Figures 1, 2a, 2b, and 8, in a first embodiment of the device of the present invention, the body (10) for the anchoring the filamentous structure is conformed as a tweezer (13, 13', 12, 11) comprising a first flat arm (13) and a second flat arm (13'), said arms (13, 13') being joined to each other by means of the capstan (12).

The porous portion (13, 13'), moreover, comprises at least a first porous zone and a second porous zone. The difference between the porosity of the first porous zone and the porosity of the second porous zone being such as to ensure a gradient of deformability. More specifically, the first porous zone, configured for the cartilage/tendon/ligament interface. The difference between the first porous zone and the second porous zone being comprised between 1% and 98%. The first porous zone (13, 13') has a porosity comprised between 2% and 90% and a pore size of 0.5 µm and 700 µm. In such preferred embodiment the pore size of the second zone is 10 µm and 900 µm. The difference of porosity can be along the longitudinal axis (Y) of the arms (13, 13') and/or along a transversal direction (X), orthogonal to the longitudinal axis (Y) of the arms (13, 13'). The structure of the porous portion (13, 13') follows a trabecular Voronoi tessellation (300) projected onto the surface of the arms (13, 13') of the tweezer (10). The latter can be made of a bioresorbable and/or inert material, in case the device is used as a device for interfacing a filamentous structure with a real biological tissue (e.g., in vivo bone tissue), or made of an inert and/or conductive material, in case the device is used as a device for interfacing a filamentous structure with a simulated biological tissue (e.g., simulated tissue of a prosthesis actuator).

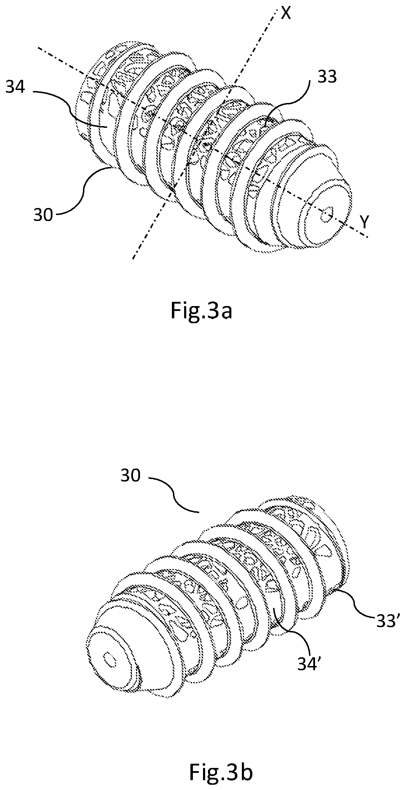

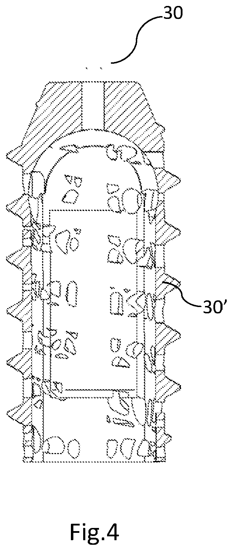

With reference to Figures 1, 2a, 2b, 3a, 3b, 4, 5a, 5b and 8, in a second embodiment thereof, the device of the present invention, comprises also a second hollow body (30) comprising at least one porous portion (33) having a trabecular structure (300), the body (10) for anchoring the filamentous structure being housed, and in particular embedded, within (30') the second body (30). The trabecular structure of the second body (30) also follows a Voronoi trabecular tessellation (300) projected onto the surface of the body (30). The second body (30) is shaped like a threaded screw and has two porous portions (33, 33') and two nonporous portions (34, 34'). Both porous portions (33, 33') of the screw (30) also, like the porous portions (13, 13') of the tweezer (10), comprise a first porous zone for the cartilage/tendon/ligament interface and a second porous zone, for the bone interface, the difference between the porosity of the first porous zone and the porosity of the second porous zone being such that an adequate gradient of deformability is generated. The difference between the first porous zone and the second porous zone being comprised between 1% and 98%. The first porous zone has a porosity comprised between 2% and 90% and a pore size of 0.5 µm and 700 µm. In such preferred embodiment the pore size of the second zone is 10 µm and 900 µm. The difference of porosity can be along the longitudinal axis (Y) of the screw (30) and/or along a transversal direction (X), orthogonal to the longitudinal axis (Y) of the screw (30). Finally, the porosity of the porous portions (33, 33') of the screw (30) may be less in the area of the screw (30) in the proximity of the capstan (12) of the tweezer (10) than the porosity in the area of the screw (30) farthest from the capstan (12) and, that is, in the proximity of the tip of the screw (30) itself.

The screw (30) may also be made of a bioresorbable and/or inert material, or made of an inert and/or conductive material depending on the application, as already described above with respect to the first embodiment of the present device. Such materials may be in particular: polyesters, polyurethanes, polyanhydrides, polycarbonates, polyamides, polyolefins and fluorinated polymers and copolymers thereof, materials of natural origin, for example polysaccharides, proteins, polyesters, polypeptides, and copolymers thereof, and/or mixtures of these materials and/or metallic materials and/or ceramic materials or combinations thereof. Moreover, the material of the screw and/or of the deformable inner element may advantageously be loaded and/or functionalized with organic and/or inorganic components capable of performing a biological function and/or modifying the physical-chemical and/or mechanical properties of the screw and/or of the deformable inner element.

In a third embodiment of the device of the present invention the body (10) for anchoring the filamentous structure comprises the body (10) for the anchoring the filamentous structure is conformed as a tweezer (13, 13', 12, 11) comprising a first flat arm (13) and a second flat arm (13'), said arms (13, 13') being joined to each other by means of the capstan (12).

The device comprises also a second hollow body (30) comprising at least one porous portion (33) having a trabecular structure (300), the body (10) for anchoring the filamentous structure being housed, and in particular embedded, within (30') the second body (30). The trabecular structure of the second body (30) also follows a Voronoi trabecular tessellation (300) projected onto the surface of the body (30). The second body (30) is shaped like a threaded screw and has two porous portions (33, 33') and two nonporous portions (34, 34'). Both the tweezer (10) and the screw (30) are each characterized by a homogeneous porosity, but have different porosity with respect to each other. In other words, the porous portions (33, 33') of the screw (30) have a porosity that differs with the porosity of the tweezer (10) such that an appropriate gradient of deformability is generated. Specifically, the porosity of the first body (10), configured for the cartilage/tendon/ligament interface, is comprised between 2% and 90% and the pore size between 10 µm and 900 µm. The difference between the porosity of the first body (10) and the porosity of the second body (30) varies between 1% and 98%. More preferably, the difference between the porosity of the first body (10) and the porosity of the second body (30) is comprised in the range between 2% and 90%.

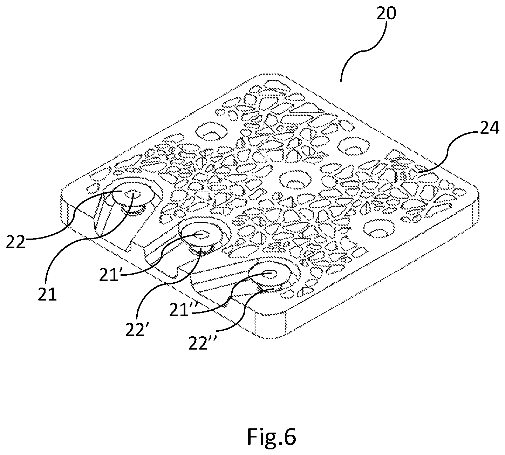

The absolute porosity value and the pore size can vary, within the ranges as defined in the claims, according to the anatomical district wherein the device is directed to be implanted and according to the clinical characteristic of the patients (e.g. it is well known that the age can affect also greatly the porosity of the bone). The difference of porosity can be along the longitudinal axis (Y) of the arms (13, 13') and/or along a transversal direction (X), orthogonal to the longitudinal axis (Y) of the arms (13, 13').With reference to Figures 6, 7a, 7b and 8, in a sixth embodiment thereof, the device of the present invention, has the form of a plate provided with a plurality of capstans (22, 22', 22"). At the center of each capstan (22, 22', 22") there may be a hole (21, 21', 21"). The porous portion (24) has a structure that follows a trabecular Voronoi tessellation (300) and comprises at least a first porous zone (24") and a second porous zone (24'). The second porous zone (24'), which is located away from the capstan (22, 22', 22") has a higher porosity than the first porous zone (24"), which is located in the proximity of the capstan (22, 22', 22"), and the difference between the porosity of the first porous zone (24") and the porosity of the second porous zone (24') is such that a gradient of deformability is provided.

More specifically, the first porous zone (24"), configured for the cartilage/tendon/ligament interface, has a porosity comprised between 2% and 90% and a pore size of 0.5 µm and 700 µm. In such preferred embodiment the pore size of the second zone is 10 µm and 900 µm. The absolute porosity value and the pore size can vary according to the anatomical district wherein the device is directed to be implanted and according to the clinical characteristic of the patients (e.g. it is well known that the age can affect also greatly the porosity of the bone). The target application (i.e biological tissue or simulated tissue) can also influence the absolute porosity and the pore size values. The difference of porosity can be along the longitudinal axis (Y) of the plate (20) and/or along a transversal direction (X), orthogonal to the longitudinal axis (Y) of the plate (20).

The plate (20) may also be made of a bioresorbable and/or inert material or made of an inert and/or conductive material depending on the application.

With reference to Figures 1, 2a, 2b, 3a, 3b, 4, 5a, 5b and 8, a first embodiment of the system for regenerating, or repairing, or replacing, tendon and/or ligamentous tissue of the present invention comprises:
- the device of the present invention according to its second embodiment, namely, a tweezer (10) - screw (30) assembly as described above;
- at least one filamentary structure comprising a plurality of nanofiber assemblies obtained by electrospinning, said plurality of assemblies being arranged to form a single bundle.

The pass bundle is wrapped to the capstan (12) of the tweezer itself (10). Additional nanofiber bundles, in addition, may pass through the pores of the porous portion (13, 13') of the tweezer (10) and, possibly, also through the pores of the porous portion (33, 33') of the screw (30), as well as through the hole (11) in the center of the capstan (12). With reference to Figures 6, 7a, 7b and 8, a second embodiment of the system for regenerating, or repairing, or replacing, tendon and/or ligament tissue of the present invention comprises:
- the device of the present invention according to its sixth embodiment, namely, a plate (20) as described above;
- at least one filamentary structure comprising a plurality of nanofiber groups obtained by electrospinning, said plurality of groups being arranged to form a single bundle.

The bundle is wrapped at each capstan (22, 22', 22") of the plate (20). Additional nanofiber bundles, in addition, can pass through the pores of the porous portion (24) of the plate (24), as well as through the holes (21, 21', 21") in the center of the capstans (22, 22', 22")

Finally, both the first and second embodiments of the system of the present invention can be used to simulate tendon and/or ligamentous tissue and thus be part of a prosthetic actuator or robotic system.

### EXAMPLES

### Finite Element Simulation

In order to have a validation of the designed device and to study the gradient of deformability the tweezer-screw assembly, a finite element simulation was performed using Ansys Workbench 2019 R3 software. Geometric CAD models were imported from SolidWorks with some simplifications on the geometry. Due to the difficulties of the meshing operation, after verifying the condition of the screw as nearly unloaded, it was decided to neglect the trabecular model.

To load the tweezer-screw assembly as in an operational working condition, a tendon-inspired system was modeled as a simplified nylon bundle assembly. To simplify the model in this simulation, only the nylon side (the working part of the biphasic bundle attachment) was considered. The bundle assembly was modeled using a hyperelastic Nylon material. The geometry of the bundles was based on a cylinder having, at the end, a ring, which surrounds the tweezer inside the screw. The main section of the cylindrical bundle has a diameter of 3.80 mm before splitting into two half-bundles. In order to have a constant cross section for the entire bundle, the half-dashes were modeled as having an elliptical cross section with a minor and major semi-axis of 1.20 mm and 1.50 mm, respectively. The two half-dashes surround the tweezer and then join together in the main circular section.

In addition, a baseplate was introduced as a support for the assembly. PLA plastic (material present in the Ansys library, *E* = 3.5 *GPa* and *σY* = 54.1 *MPa*) was used as the material of the base and the tweezer in the simulation. Considering the absence of the trabecular model, reduced properties were assigned to the screw, to confer greater strain, with a Young's modulus of 2.4 *GPa.*

The baseplate was used as a fixed support to which the Voronoi porosity screw is attached, allowing, thus testing the thread resistance under load. A load was applied to the opposite side of the tweezer cross-section. To simplify the finite element simulation, a quarter of the CAD model was used. This simplification was done everywhere less than where the holes on the tweezer and the screw did not exhibit true symmetries. This assumption was imposed to significantly reduce the computational time.

The model was subjected to a "meshing" procedure using the Ansys Tetrahedra method. Tetrahedra with a maximum size of 0.40 mm were used for meshing the tweezer and the trabecular screw. To better study the trabecular surface subjected to higher loading, tetrahedral "meshes" with a maximum size of 0.25 mm were used for the tweezer. For the baseplate, tetrahedral "meshes" with a maximum dimension of 0.50 mm were used. The entire "mesh" of the model has 207402 nodes and 134632 elements, with a "meshing" quality of 0.82. Only linear elements were used.

Two different contacts were used to better simulate the interaction between the four parts:
- friction with a coefficient of friction = 0.3, between the base and the screw in the threaded connection area, and, between the screw and the tweezer, to simulate interference conditions;
- friction with a coefficient of friction = 0.4, between the tweezer and the bundle assembly, on the faces where wrapping occurred.

Contact between the trabecular screw and the tweezer was imposed in the area where the groove pitch provides axial clamping of the tweezer and between the inside of the screw and the top face of the tweezer. Lateral contact was imposed to prevent instability during compression of the tweezer arms. The model was clamped using a fixed support on the bottom face of the base. The load was applied as a force on the upper section of the bundle, simulating the force applied by the connected muscle-inspired bundle.

### Simulation Results

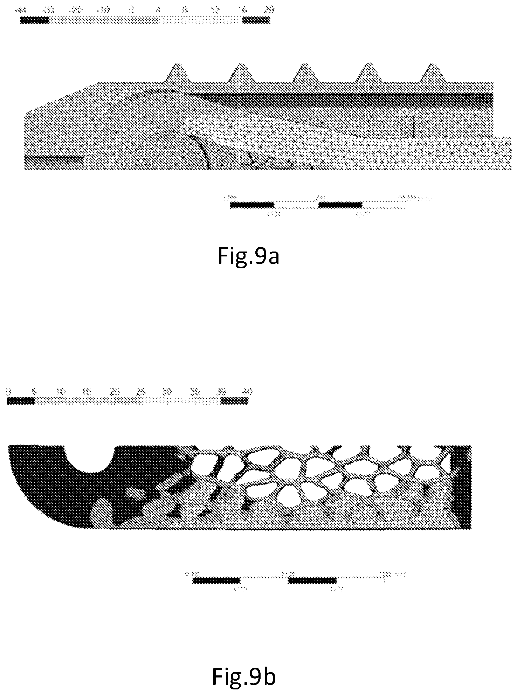

The finite element simulation was performed in 2 hours and 24 minutes. Due to the risk of elastic instability for a fully trabecular tweezer, a partial trabecular tweezer was used in this simulation. This tweezer exhibited compressive loading but no signs of impending instability. The normal stress results are shown in Figure 9a.

As imagined, the normal stress presents the highest value at the point where there is detachment between the bundle assembly and the capstan, due to a concentration of stresses. A normal stress concentration factor of 3.24 can be calculated.

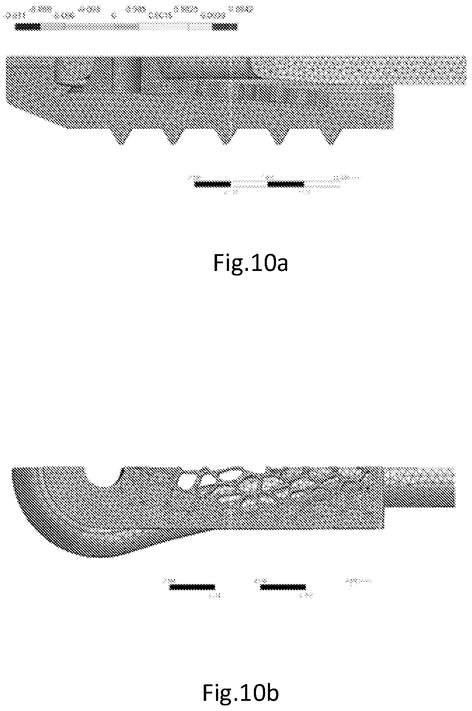

The normal strain analysis presented a high strain of the bundle assembly, while the trabecular screw proved to be almost non-deformable. The tweezer, on the other hand, presented an average strain compared to that of the other two components (screw and bundle assembly), thus providing for the desired strain gradient of the bioinspired junction. The normal strains of the tweezer and the bundle assembly are shown in more detail in Figures 10a and 10b.

## Claims

1. Device for interfacing at least one filamentous structure, with real or simulated biological tissue, comprising at least one body (10, 20) for anchoring the filamentous structure, said device being **characterized in that** said body (10, 20) comprises:
- at least one capstan (12, 22, 22") configured for the wrapping of the filamentous structure; and
- at least one porous portion (13, 13', 24, 24', 24") having a trabecular structure (300) and including at least a first porous zone (13, 13', 24") in the proximity of the capstan (12, 22, 22') having a porosity comprised between 2% and 90% and a pore size comprised between 0.5 µm and 700 µm, and a second porous zone (24'), farther from the capstan (12, 22, 22') than the first porous zone (24') is, the second porous zone (24') having a pore size comprised between 10 µm and 900 µm, and the difference between the porosity of first porous zone (13, 13', 24") and the porosity of the second porous zone (24') being comprised between 1% and 98%, said difference being such to ensure a gradient of deformability at least between the first porous zone (13, 24") and the second porous zone (24').

2. Device for interfacing at least one filamentous structure, with real or simulated biological tissue, comprising:
- a first body (10) for anchoring the filamentous structure, said first body (10) comprising:
• at least one capstan (12) configured for the wrapping of the filamentous structure, and
• at least one porous portion (13, 13') having a trabecular structure (300); and
- a second hollow body (30) including at least one porous portion (33) having a trabecular structure (300), the body (10) for anchoring the filamentous structure being housed inside (30') of the second body (30);
said device being **characterized in that**:
- the first body (10) is conformed as a tweezer (13, 13', 12, 11) comprising a first flat arm (13) and a second flat arm (13'); and **in that**
- the at least one porous portion (13, 13') of the first body (10) having a porosity comprised between 2% and 90% and a pore size comprised between 0.5 µm and 700 µm, the at least one porous portion (33) of the second body (30) having a pore size comprised between 10 µm and 900 µm and the difference between the porosity of at least one porous portion (13, 13') of the first body (10) and the porosity of the at least one porous portion (33) of the second body (30) being comprised between 2% and 90% and said difference being such to ensure a gradient of deformability at least between the first body (10) and the second body (30).

3. Device according to claim 1, wherein the structure of the at least one porous portion (13, 13', 24, 24', 24") follows a Voronoi tesseletion (300) projected onto the surface of the body (10, 20) for anchoring the filamentous structure.

4. Device according to claim 1, wherein the body (10) for the anchoring the filamentous structure is conformed as a tweezer (10) comprising a first flat arm (13) and a second flat arm (13'), said arms (13, 13') being joined to each other by means of the capstan (12).

5. Device according to claim 1 or 3, wherein the body (20) has the shape of a plate (20) provided with a plurality of capstans (22, 22', 22").

6. Device according to claim 2, wherein the at least one porous portion (33) of the second body (30) comprises a first porous zone in the proximity of the capstan (12) having a porosity comprised between 2% and 90% and a pore size comprised between 0.5 µm and 700 µm and a second porous zone farther from the capstan (12, 22, 22') than the first porous zone (24') is, the pore size of the second porous zone being comprised between 10 µm and 900 µm and the difference between the porosity of the first porous zone and the porosity of the second porous zone being comprised between 1% and 98%.

7. Device according to claim 4, wherein the body (10) for anchoring the filamentous structure is embedded inside the second body (30).

8. Device according to claim 7, wherein the second body (30) has the shape of a threaded screw and the difference of porosity is along a longitudinal axis (Y) of the screw (3) and/or along a transversal direction (X) orthogonal to the longitudinal axis (Y) of the screw (30).

9. Device according to claim 7 or 8, wherein the at least one porous portion (13, 13') of the tweezer (10) comprises a first porous zone in the proximity of the capstan (12) having a porosity comprised between 2% and 90% and a pore size comprised between 0.5 µm and 700 µm and a second porous zone with a pore size being comprised between 10 µm and 900 µm, the difference between the porosity of the first porous zone and the porosity of the second porous zone of the first body (10) being comprised between 1% and 98%.

10. Device according to the preceding claim, wherein the difference of porosity of the first porous zone and the porosity of the second porous zone of the tweezer (10) is along a longitudinal axis (Y) of the arms (13, 13') of the tweezer (10) and/or along a transversal direction (X) orthogonal to the longitudinal axis (Y) of the arms (13, 13') of the tweezer (10).

11. Device according to any of the claims from 1 to 10 wherein at least one part of the device is made of at least one bioreabsorbable and/or biocompatible and/or inert and/or conductive material.

12. Device according to the previous claim wherein said at least one material is selected from the group consisting of: polyesters, polyurethanes, polyanhydrides, polycarbonates, polyamides, polyolefins, fluorinated polymers, polyester copolymers, polyurethane copolymers, polyanhydrides copolymers, polycarbonates copolymers, polyamide copolymers, polyolefin copolymers, fluorinated polymer copolymers, polysaccharides, proteins, polyesters, polypeptides, polysaccharide copolymers, protein copolymers, polyester copolymers, polypeptide copolymers, metal and ceramic material.

13. System for regeneration, or repair, or replacement, or simulation of tendon and/or ligament tissue including:
- a device according to any of the claims from 1 to 12; and
- at least one filamentous structure comprising a plurality of electrospun nanofibre groups, said plurality of groups being arranged to form a single bundle; said bundle being wrapped to the capstan (12).

14. System according to claim 13, wherein the nanofiber bundle passes into the pores of the porous portion (13, 13', 24).

15. Prosthetic actuator or robotic system including a system according to claim 13 and 14.

## Patentansprüche

1. Vorrichtung zur Verbindung mindestens einer fadenförmigen Struktur mit echtem oder simuliertem biologischem Gewebe, umfassend mindestens einen Körper (10, 20) zur Verankerung der fadenförmigen Struktur, wobei die Vorrichtung **dadurch gekennzeichnet ist, dass** der Körper (10, 20) Folgendes umfasst:
- mindestens eine Winde (12, 22, 22"), die zum Aufwickeln der fadenförmigen Struktur konfiguriert ist; und
- mindestens einen porösen Abschnitt (13, 13', 24, 24', 24"), der eine trabekuläre Struktur (300) aufweist und mindestens eine erste poröse Zone (13, 13', 24") umfasst in der Nähe der Winde (12, 22, 22'), die eine Porosität zwischen 2% und 90% und einer Porengröße zwischen 0,5 µm und 700 µm aufweist, und eine zweite poröse Zone (24'), die weiter von der Winde (12, 22, 22') entfernt ist als die erste poröse Zone (24'), wobei die zweite poröse Zone (24') eine Porengröße zwischen 10 µm und 900 µm aufweist und die Differenz zwischen der Porosität der ersten porösen Zone (13, 13', 24") und der Porosität der zweiten porösen Zone (24') zwischen 1% und 98% liegt, wobei die besagte Differenz so ist, dass sie einen Verformbarkeitsgradienten zumindest zwischen der ersten porösen Zone (13, 24") und der zweiten porösen Zone (24') gewährleistet.

2. Vorrichtung zur Verbindung mindestens einer fadenförmigen Struktur mit echtem oder simuliertem biologischem Gewebe, Folgendes umfassend:
- einen ersten Körper (10) zur Verankerung der fadenförmigen Struktur, wobei der besagte erste Körper (10) Folgendes umfasst:
• mindestens eine Winde (12), die zum Aufwickeln der fadenförmigen Struktur konfiguriert ist, und
• mindestens einen porösen Abschnitt (13, 13'), der eine trabekuläre Struktur (300) aufweist; und
- einen zweiten hohlen Körper (30), der mindestens einen porösen Abschnitt (33) umfasst, der eine trabekuläre Struktur (300) aufweist, wobei der Körper (10) zur Verankerung der faserförmigen Struktur im Inneren (30') des zweiten Körpers (30) untergebracht ist;
wobei die besagte Vorrichtung **dadurch gekennzeichnet ist, dass**:
- der erste Körper (10) als Pinzette (13, 13', 12, 11) ausgebildet ist, die einen ersten flachen Arm (13) und einen zweiten flachen Arm (13') umfasst; und dadurch, dass
- der mindestens eine poröse Abschnitt (13, 13') des ersten Körpers (10) eine Porosität aufweist zwischen 2% und 90% und eine Porengröße zwischen 0,5 µm und 700 µm, wobei der mindestens eine poröse Abschnitt (33) des zweiten Körpers (30) eine Porengröße zwischen 10 µm und 900 µm aufweist und die Differenz zwischen der Porosität des mindestens einen porösen Abschnitts (13, 13') des ersten Körpers (10) und der Porosität des mindestens einen porösen Abschnitts (33) des zweiten Körpers (30) zwischen 2% und 90% liegt und die besagte Differenz so ist, dass sie einen Verformbarkeitsgradienten zumindest zwischen dem ersten Körper (10) und dem zweiten Körper (30) gewährleistet.

3. Vorrichtung gemäß Anspruch 1, wobei die Struktur des mindestens einen porösen Abschnitts (13, 13', 24, 24', 24") einer auf die Oberfläche des Körpers (10, 20) projizierten Voronoi-Tessellation (300) folgt, um die fadenförmige Struktur zu verankern.

4. Vorrichtung gemäß Anspruch 1, wobei der Körper (10) zur Verankerung der fadenförmigen Struktur als Pinzette (10) ausgebildet ist, die einen ersten flachen Arm (13) und einen zweiten flachen Arm (13') umfasst, wobei die besagten Arme (13, 13') mittels der Winde (12) miteinander verbunden sind.

5. Vorrichtung gemäß Anspruch 1 oder 3, wobei der Körper (20) die Form einer Platte (20) aufweist, die mit einer Vielzahl von Winden (22, 22', 22") versehen ist.

6. Vorrichtung gemäß Anspruch 2, wobei der mindestens eine poröse Abschnitt (33) des zweiten Körpers (30) eine erste poröse Zone umfasst in der Nähe der Winde (12), die eine Porosität aufweist zwischen 2% und 90% und eine Porengröße zwischen 0,5 µm und 700 µm, und eine zweite poröse Zone, die weiter von der Winde (12, 22, 22') entfernt ist als die erste poröse Zone (24'), wobei die Porengröße der zweiten porösen Zone zwischen 10 µm und 900 µm liegt und die Differenz zwischen der Porosität der ersten porösen Zone und der Porosität der zweiten porösen Zone zwischen 1% und 98% liegt.

7. Vorrichtung gemäß Anspruch 4, wobei der Körper (10) zur Verankerung der fadenförmigen Struktur innerhalb des zweiten Körpers (30) eingebettet ist.

8. Vorrichtung gemäß Anspruch 7, wobei der zweite Körper (30) die Form einer Gewindeschraube aufweist und die Differenz der Porosität entlang einer Längsachse (Y) der Schraube (3) und/oder entlang einer Querrichtung (X) orthogonal zur Längsachse (Y) der Schraube (30) verläuft.

9. Vorrichtung gemäß Anspruch 7 oder 8, wobei der mindestens eine poröse Abschnitt (13, 13') der Pinzette (10) eine erste poröse Zone umfasst in der Nähe der Winde (12), die eine Porosität aufweist zwischen 2% und 90% und eine Porengröße zwischen 0,5 µm und 700 µm, und eine zweite poröse Zone mit einer Porengröße zwischen 10 µm und 900 µm, wobei die Differenz zwischen der Porosität der ersten porösen Zone und der Porosität der zweiten porösen Zone des ersten Körpers (10) zwischen 1% und 98% liegt.

10. Vorrichtung gemäß dem vorhergehenden Anspruch, wobei die Differenz zwischen der Porosität der ersten porösen Zone und der Porosität der zweiten porösen Zone der Pinzette (10) entlang einer Längsachse (Y) der Arme (13, 13') der Pinzette (10) verläuft und/oder entlang einer Querrichtung (X) orthogonal zur Längsachse (Y) der Arme (13, 13') der Pinzette (10).

11. Vorrichtung gemäß einem jeden der vorhergehenden Ansprüche von 1 bis 10, wobei mindestens ein Teil der Vorrichtung aus mindestens einem bioresorbierbaren und/oder biokompatiblen und/oder inerten und/oder leitfähigen Material besteht.

12. Vorrichtung gemäß dem vorhergehenden Anspruch, wobei das besagte mindestens eine Material aus der Gruppe ausgewählt ist, bestehend aus: Polyestern, Polyurethanen, Polyanhydriden, Polycarbonaten, Polyamiden, Polyolefinen, fluorierten Polymeren, Polyester-Copolymeren, Polyurethan-Copolymeren, Polyanhydrid-Copolymeren, Polycarbonat-Copolymeren, Polyamid-Copolymeren, Polyolefin-Copolymeren, fluorierten Polymer-Copolymeren, Polysacchariden, Proteinen, Polyestern, Polypeptiden, Polysaccharid-Copolymeren, Protein-Copolymeren, Polyester-Copolymeren, Polypeptid-Copolymeren, Metall- und Keramikmaterialien.

13. System zur Regeneration, Reparatur, zum Ersatz oder zur Simulation von Sehnen- und/oder Bändergewebe, umfassend:
- eine Vorrichtung gemäß einem jeden der Ansprüche 1 bis 12; und
- mindestens eine fadenförmige Struktur, umfassend eine Vielzahl von Gruppen elektrogesponnener Nanofasern, wobei die besagte Vielzahl von Gruppen angeordnet ist, um ein einziges Bündel zu bilden; wobei das besagte Bündel um die Winde (12) gewickelt ist.

14. System gemäß Anspruch 13, wobei das Nanofaserbündel in die Poren des porösen Abschnitts (13, 13', 24) eindringt.

15. Prothetischer Aktuator oder Robotersystem, umfassend ein System gemäß Anspruch 13 und 14.

## Revendications

1. Dispositif pour interfacer au moins une structure filamenteuse avec un tissu biologique réel ou simulé, comprenant au moins un corps (10, 20) pour ancrer la structure filamenteuse, ledit dispositif étant **caractérisé par le fait que** ledit corps (10, 20) comprend :
- au moins un cabestan (12, 22, 22") configuré pour l'enroulement de la structure filamenteuse ; et
- au moins une portion poreuse (13, 13', 24, 24', 24") ayant une structure trabéculaire (300) et comprenant au moins une première zone poreuse (13, 13', 24") à proximité du cabestan (12, 22, 22') ayant une porosité comprise entre 2 % et 90 % et une taille de pore comprise entre 0,5 µm et 700 µm, et une deuxième zone poreuse (24'), plus éloignée du cabestan (12, 22, 22') que ne l'est la première zone poreuse (24'), la deuxième zone poreuse (24') ayant une taille de pore comprise entre 10 µm et 900 µm, et la différence entre la porosité de la première zone poreuse (13, 13', 24") et la porosité de la deuxième zone poreuse (24') étant comprise entre 1 % et 98 %, ladite différence étant telle qu'elle assure un gradient de déformabilité au moins entre la première zone poreuse (13, 24") et la deuxième zone poreuse (24').

2. Dispositif pour interfacer au moins une structure filamenteuse avec un tissu biologique réel ou simulé, comprenant :
- un premier corps (10) pour ancrer la structure filamenteuse, ledit premier corps (10) comprenant :
• au moins un cabestan (12) configuré pour l'enroulement de la structure filamenteuse, et
• au moins une portion poreuse (13, 13') ayant une structure trabéculaire (300) ; et
- un deuxième corps creux (30) comprenant au moins une portion poreuse (33) ayant une structure trabéculaire (300), le corps (10) pour ancrer la structure filamenteuse étant logé à l'intérieur (30') du deuxième corps (30) ;
ledit dispositif étant **caractérisé par le fait que** :
- le premier corps (10) est conformé en pince (13, 13', 12, 11) comprenant un premier bras plat (13) et un deuxième bras plat (13') ; et **par le fait que**
- l'au moins une portion poreuse (13, 13') du premier corps (10) ayant une porosité comprise entre 2 % et 90 % et une taille de pore comprise entre 0,5 µm et 700 µm, l'au moins une portion poreuse (33) du deuxième corps (30) ayant une taille de pore comprise entre 10 µm et 900 µm et la différence entre la porosité de l'au moins une portion poreuse (13, 13') du premier corps (10) et la porosité de l'au moins une portion poreuse (33) du deuxième corps (30) étant comprise entre 2 % et 90 % et ladite différence étant telle qu'elle assure un gradient de déformabilité au moins entre le premier corps (10) et le deuxième corps (30).

3. Dispositif selon la revendication 1, où la structure de l'au moins une portion poreuse (13, 13', 24, 24', 24") suit une tessellation de Voronoï (300) projetée sur la surface du corps (10, 20) pour ancrer la structure filamenteuse.

4. Dispositif selon la revendication 1, où le corps (10) pour ancrer la structure filamenteuse est conformé en pince (10) comprenant un premier bras plat (13) et un deuxième bras plat (13'), lesdits bras (13, 13') étant reliés l'un à l'autre par l'intermédiaire du cabestan (12).

5. Dispositif selon la revendication 1 ou 3, où le corps (20) a la forme d'une plaque (20) pourvue d'une multitude de cabestans (22, 22', 22").

6. Dispositif selon la revendication 2, où l'au moins une portion poreuse (33) du deuxième corps (30) comprend une première zone poreuse à proximité du cabestan (12) ayant une porosité comprise entre 2 % et 90 % et une taille de pore comprise entre 0,5 µm et 700 µm et une deuxième zone poreuse plus éloignée du cabestan (12, 22, 22') que ne l'est la première zone poreuse (24'), la taille de pore de la deuxième zone poreuse étant comprise entre 10 µm et 900 µm et la différence entre la porosité de la première zone poreuse et la porosité de la deuxième zone poreuse étant comprise entre 1 % et 98 %.

7. Dispositif selon la revendication 4, où le corps (10) pour ancrer la structure filamenteuse est intégré à l'intérieur du deuxième corps (30).

8. Dispositif selon la revendication 7, où le deuxième corps (30) a la forme d'une vis filetée et la différence de porosité est orientée selon un axe longitudinal (Y) de la vis (30) et/ou selon une direction transversale (X) orthogonale à l'axe longitudinal (Y) de la vis (30).

9. Dispositif selon la revendication 7 ou 8, où l'au moins une portion poreuse (13, 13') de la pince (10) comprend une première zone poreuse au voisinage du cabestan (12) ayant une porosité comprise entre 2 % et 90 % et une taille de pore comprise entre 0,5 µm et 700 µm et une deuxième zone poreuse ayant une taille de pore comprise entre 10 µm et 900 µm, la différence entre la porosité de la première zone poreuse et la porosité de la deuxième zone poreuse du premier corps (10) étant comprise entre 1 % et 98 %.

10. Dispositif selon la revendication précédente, où la différence de porosité de la première zone poreuse et de la porosité de la deuxième zone poreuse de la pince (10) est orientée selon un axe longitudinal (Y) des bras (13, 13') de la pince (10) et/ou selon une direction transversale (X) orthogonale à l'axe longitudinal (Y) des bras (13, 13') de la pince (10).

11. Dispositif selon l'une des revendications 1 à 10, où au moins une partie du dispositif est fabriquée en au moins un matériau biorésorbable et/ou biocompatible et/ou inerte et/ou conducteur.

12. Dispositif selon la revendication précédente, où ledit au moins un matériau est sélectionné dans le groupe constitué de : polyesters, polyuréthanes, polyanhydrides, polycarbonates, polyamides, polyoléfines, polymères fluorés, copolymères de polyesters, copolymères de polyuréthanes, copolymères de polyanhydrides, copolymères de polycarbonates, copolymères de polyamides, copolymères de polyoléfines, copolymères de polymères fluorés, polysaccharides, protéines, polyesters, polypeptides, copolymères de polysaccharides, copolymères de protéines, copolymères de polyesters, copolymères de polypeptides, matériaux métalliques et céramiques.

13. Système pour la régénération, ou la réparation, ou le remplacement, ou la simulation de tissu tendineux et/ou ligamentaire comprenant :
- un dispositif selon l'une des revendications 1 à 12 ; et
- au moins une structure filamenteuse comprenant une multitude de groupes de nanofibres électrofilées, ladite multitude de groupes étant agencée de manière à former un faisceau unique ; ledit faisceau étant enroulé autour du cabestan (12).

14. Système selon la revendication 13, où le faisceau de nanofibres pénètre dans les pores de la portion poreuse (13, 13', 24).

15. Actionneur prothétique ou système robotique comprenant un système selon les revendications 13 et 14.
